(19) Europäisches Patentamt
European Patent Office
Office européen des brevets

(11) Veröffentlichungsnummer: **0 167 489**
**B1**

(12) # EUROPÄISCHE PATENTSCHRIFT

(45) Veröffentlichungstag der Patentschrift: **25.01.89**

(51) Int. Cl.⁴: **C 07 D 335/16**, C 08 F 2/50, G 03 C 1/68

(21) Anmeldenummer: **85810300.5**

(22) Anmeldetag: **28.06.85**

(54) **Flüssige Thioxanthoncarbonsäureester.**

(30) Priorität: **04.07.84 CH 3228/84**

(43) Veröffentlichungstag der Anmeldung: **08.01.86 Patentblatt 86/2**

(45) Bekanntmachung des Hinweises auf die Patenterteilung: **25.01.89 Patentblatt 89/4**

(84) Benannte Vertragsstaaten: **BE DE FR GB IT NL SE**

(56) Entgegenhaltungen: **EP-A-0 033 720**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Fischer, Walter, Dr., Vogesenstrasse 77, CH- 4153 Reinach (CH)**
Erfinder: **Berner, Godwin, Dr., Sommerhalde 5, CH- 4102 Binningen (CH)**

EP 0 167 489 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft flüssige Thioxanthoncarbonsäureester mit Polyoxaalkylenresten in der Estergruppe, Mischungen solcher Ester mit einem organischen Amin oder mit einem radikalischen oder kationischen Photoinitiator, sowie photopolymerisierbare Systeme, die solche Mischungen enthalten.

In der DE-A1-3 228 371 sind Alkylthioxanthone als Photoinitiatoren für UV-härtbare Harzsysteme beschrieben. Sie liegen in flüssiger Form vor und können dadurch in Präpolymerformulierungen dispergiert werden. Diese Alkylthioxanthone konnten sich wegen toxikologischer Bedenken nicht durchsetzen.

In der DE-A1-3 018 891 sind Thioxanthoncarbonsäureester als Photoinitiatoren beschrieben. Unter den kristallinen Verbindungen ist auch der als Öl vorliegende Ester aus Triäthylenglykolmonomethylether und Thioxanthon-1-carbonsäure erwähnt. Es hat sich nachträglich herausgestellt, dass die Verbindung nach einiger Zeit kristallisiert. Neben einer unzureichenden Lagerstabilität ist hiermit auch ein erheblicher Nachteil für die Produktion verbunden. Wenn sich während der Produktion Kristallkeime bilden, können diese nicht mehr aus den Apparaturen entfernt werden, was zur Kristallisation der gewünschten flüssigen Verbindungen und damit zur Einstellung der Produktion führen kann.

In der EP-A1-0 033 720 sind Thioxanthoncarbonsäureester mit funktionellen Estergruppen beschrieben. Bei den erwähnten Diethylenglykol- und Triethylenglykolestern der 7-Methylthioxanthon-3-carbonsäure handelt es sich um feste Verbindungen.

Flüssige Thioxanthone können besser in strahlungshärtbare Systeme eingearbeitet und verteilt werden. Zusätzlich sind solche Thioxanthone wünschenswert, die in flüssigen, besonders wässrigen Lackformulierungen dispergiert bzw. emulgiert werden können.

Gegenstand vorliegender Erfindung sind flüssige Thioxanthoncarbonsäureester Der Formel I und flüssige Mischungen von Thioxanthoncarbonsäureestern der Formel I

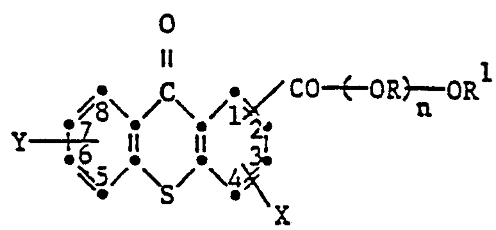

(I) ,

worin

X H, Halogen, $NO_2$, COOH, gegebenenfalls alkylsubstituiertes Amino, $C_1$-$C_{20}$-Alkyl, $C_1$-$C_{20}$-Alkoxy, $C_1$-$C_{20}$-Alkylthio, $C_1$-$C_{20}$-Alkylsulfinyl $C_1$-$C_{20}$-Alkylsulfonyl-, unsubstituiertes oder substituiertes Phenoxy, Phenylthio, Phenylsulfinyl oder Phenylsulfonyl oder -COOR$^a$ ist, worin

R$^a$ für $C_1$-$C_6$-Alkyl, Cyclopentyl, Cyclohexyl, Phenyl, ($C_1$-$C_4$-Alkyl)-Phenyl, Benzyl oder ($C_1$-$C_4$-Alkyl)-Benzyl steht,

Y unabhängig die gleiche Bedeutung wie X hat,

n für eine Zahl von 4 bis 25 steht,

R lineares oder verzweigtes $C_2$-$C_6$-Alkylen bedeutet, und

R$^1$ für H, $C_1$-$C_{12}$-Alkyl oder einen Rest der Formel II

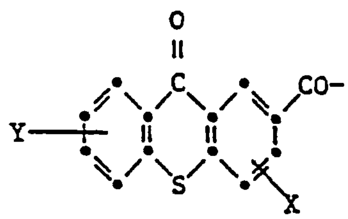

(II)

steht wobei n eine Zahl von 7 - 25 ist wenn R Ethylen und R$^1$ H bedeuten, und n eine Zahl von 11 - 25 ist, wenn R für Ethylen und R$^1$ für Methyl stehen.

Unter den Mischungen sind solche mit Verbindungen der Formel I bevorzugt, worin n verschiedene Zahlen bedeutet, oder Mischungen solcher Verbindungen der Formel I, worin zum einen R$^1$ für H steht und R$^1$ zum anderen für einen Rest der Formel II steht, wobei in diesen Mischungen zusätzlich Verbindungen der Formel I zugegen sein können, worin n verschiedene Zahlen bedeutet.

X und Y als Halogen sind bevorzugt Brom und besonders Chlor oder Fluor. X und Y als gegebenenfalls mit Alkyl substituiertes Amino kann $-NH_2$, $-NHR^2$ oder $-NR^2_2$ sein, worin R$^2$ lineares oder verzweigtes Alkyl mit bevorzugt 1 bis 6 C-Atomen bedeutet. Beispiele für Alkyl sind Methyl, Ethyl, n-Propyl, i-Propyl, Butyl, Pentyl und Hexyl.

X und Y als Alkyl und der Alkylrest in den Alkoxy-, Alkylthio-, Alkylsulfinyl- und Alkylsulfonylgruppen für X und Y kann linear oder verzweigt sein und vorzugsweise 1 bis 12, besonders 1 bis 6 und insbesondere 1 bis 4 C-Atome enthalten. Beispiele sind: Methyl, Ethyl, n-Propyl, i-Propyl, n-Butyl, i-Butyl, t-Butyl, Pentyl, Hexyl, Heptyl, Octyl, 2-Äthylhexyl, Nonyl, Decyl, Undecyl und Dodecyl. X und Y als Alkyl ist besonders Methyl.

Geeignete Substituenten für die Phenoxy- Phenylthio-, Phenylsulfonyl- und Phenylsulfinylgruppen sind beispielsweise $C_1$-$C_4$-Alkyl und Halogen wie F, Cl oder Br.

$R^a$ enthält als Alkyl 1 bis 6 C-Atome und ist besonders Methyl oder Ethyl.

X steht bevorzugt für H. Der Rest Y ist bevorzugt in 7-Stellung gebunden. In einer bevorzugten Ausführungsform ist Y H oder $C_1$-$C_6$-Alkyl, besonders Methyl.

Die -CO-(-OR-)$_n$-$OR^1$ Gruppe ist bevorzugt in 1- oder 3-Stellung gebunden.

Beispiele für R als Alkylen sind Ethylen, 1,2- und 1,3-Propylen, 1,2-, 1,3- und 1,4-Butylen, 1,2-, 1,3-, 1,4- und 1,5-Pentylen und 1,2-, 1,3-, 1,4-, 1,5- und 1,6-Hexylen. Bevorzugt ist R $C_2$-$C_4$-Alkylen, besonders Ethylen, 1,2- oder 1,3-Propylen.

$R^1$ als Alkyl kann linear oder verzweigt sein und enthält 1 bis 12, besonders 1 bis 6 C-Atome. Beispiele für Alkyl sind zuvor für X erwähnt worden. In einer bevorzugten Ausführungsform steht $R^1$ für H, Methyl, Ethyl oder einen Rest der Formel II.

Wenn R lineares Alkylen ist, stellt n bevorzugt eine Zahl von 9 bis 25 dar und wenn R verzweigtes Alkylen ist, stellt n bevorzugt eine Zahl von 6 bis 25 dar.

In einer bevorzugten Ausführungsform steht n für eine Zahl von 8 bis 20, besonders 8 bis 18, insbesondere 8 bis 14, wenn $R^1$ H oder einen Rest der Formel II bedeutet. In einer anderen bevorzugten Ausführungsform ist n eine Zahl von 11 bis 20, besonders 12 bis 18, wenn R Alkyl bedeutet.

In einer besonders bevorzugten Untergruppe stehen R für Ethylen, $R^1$ für H oder einen Rest der Formel II und n für eine Zahl von 8 bis 14 oder R für Ethylen, $R^1$ für Methyl und n für eine Zahl von 12 bis 18.

Die Verbindungen der Formel I und ihre Mischungen werden nach üblichen Veresterungsverfahren durch die Umsetzung von Thioxanthoncarbonsäuren der Formel

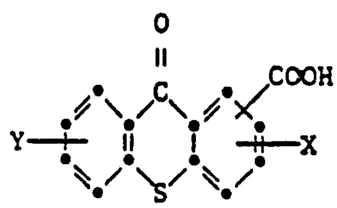

oder deren Ester bildenden Derivaten, z. B. Säureester oder Säurehalogenide, mit Alkoholen der Formel

$R^5O$-(-RO-)$_n$-H,

worin $R^5$ H oder Alkyl bedeutet, erhalten. Die Reaktion kann in Gegenwart inerter organischer Lösungsmittel und bei erhöhten Temperaturen, z. B. 50°C - 200°C, durchgeführt werden. Inerte Lösungsmittel sind z. B. aromatische Kohlenwasserstoffe wie Benzol, Toluol, Xylol, Chlorbenzol und 1,2-Dichlorbenzol.

Bei der Veresterung der Carbonsäuren bzw. Umesterung von Carbonsäureestern können Säuren oder Metallverbindungen als Katalysatoren eingesetzt werden, z. B. Salzsäure, Schwefelsäure und Sulfonsäuren, Tetraalkylorthotitanate oder Dialkylzinnoxide.

Wenn $R^5$ Alkyl ist, wird bevorzugt 1 Äquivalent Alkohol der Formel

$R^5O$-(-RO-)$_n$-H

eingesetzt. Wenn $R^5$ für H steht, können 0,5 bis 1 Äquivalent Dialkoholder Formel

HO-(-RO-)$_n$-H

eingesetzt werden. Je nach Zugabemenge erhält man dann Verbindungen der Formel I, worin $R^1$ H bzw. ein Rest der Formel II ist oder Mischungen solcher Verbindungen.

Thioxanthoncarbonsäuren und Säurederivate sind aus DE-A1-3 018 891 und EP-A1-0 033 720 bekannt oder können nach analogen Verfahren hergestellt werden. Die verwendeten Alkohole sind ebenfalls bekannt und teilweise käuflich.

Bei den Verbindungen der Formel I und deren Mischungen handelt es sich um flüssige bis ölige Substanzen, die problemlos in Substrate eingearbeitet werden können. Überraschend zeichnen sie sich auch durch eine geringe Vergilbung aus.

Die Verbindungen der Formel I und deren Mischungen können zusammen mit Wasserstoff-Donatoren, insbesondere mit organischen Aminen als Photoinitiatoren für die Photopolymerisation von ethylenisch ungesättigten Verbindungen, oder für die Photovernetzung von Polyolefinen, sowie als Photoredoxkatalysatoren z. B. für die stromlose Metallabscheidung verwendet werden. Die Erfindung betrifft daher auch Gemische aus

A) mindestens einer Verbindung der Formel I und
B) mindestens einem organischen Amin,

als Initiatoren für die Photopolymerisation ethylenisch ungesättigter Verbindungen oder für die photochemische Vernetzung von Polyolefinen.

Die verwendeten organischen Amine können aliphatische, aromatische, araliphatische, cycloaliphatische oder heterocyclische Amine sein. Sie können primäre, sekundäre oder tertiäre Amine sein.

Beispiele hierfür sind:

Butylamin, Dibutylamin, Tributylamin, Cyclohexylamin, Benzyldimethylamin, Dicyclohexylamin, Triethylamin, Phenyl-diethanolamin, Piperidin, Piperazin, Morpholin, Pyridin, Chinolin, p-Dimethylaminobenzoesäureäthylester oder Michlers Keton (4,4'-Bis-dimethylamino-benzophenon).

Beispiele für bevorzugte aliphatische tertiäre Amine sind Trimethylamin, Triethylamin, Tri-isopropylamin, Tributylamin, Dodecyl-dimethylamin, Octyl-dimethylamin, Triethanolamin, Tris(hydroxypropyl)amin, N-Methyldiethanolamin oder N-Butyl-diethanolamin.

Die erfindungsgemässen Gemische enthalten die Verbindungen der Formel I und die organischen Amine bevorzugt in einem Gewichtsverhältnis von 4 : 1 bis 1 : 4.

Photopolymerisierbare Verbindungen sind beispielsweise ungesättigte Monomere, wie Ester von Acryl- oder Methylacrylsäure, z. B. Methyl-, Ethyl, n- oder tert.-Butyl-, Isooctyl- oder Hydroxyethylacrylat, Methyl- oder Äthylmethacrylat, Ethylen-diacrylat, Butandioldiacrylat, Hexandioldiacrylat, Neopentyl-diacrylat, Trimethylolpropantrisacrylat, Pentaerythrit-tetraacrylat oder Pentaerythrit-trisacrylat; Acrylnitril, Methacrylnitril, Acrylamid, Methacrylamid, N-substituierte (Meth)-acrylamide; Vinylester, wie z. B. Vinylacetat, -propionat, -acrylat oder -succinat; sonstige Vinylverbindungen, wie Vinylether, Vinylketone, Vinylsulfone, Styrol, Alkylstyrole, Halogenstyrole, Divinylbenzol, N,N'-Divinylharnstoff, Vinylnaphthalin, N-Vinylpyrrolidon, Vinylchlorid oder Vinylidenchlorid; Allylverbindungen, wie Diallylphthalat, Diallylmaleat, Triallylisocyanurat, Triallylphosphat oder Äthylenglycol-diallyläther und die Mischungen von solchen ungesättigten Monomeren.

Besonders geeignet sind die erfindungsgemässen Gemische für die Photopolymerisation von Acrylsäureestern und deren Gemischen.

Weitere Beispiele sind ungesättigte Acrylharze. Hierzu zählen beispielsweise Umsetzungsprodukte von Polyepoxiden (Epoxidharzen) mit Acrylsäure oder Methacrylsäure oder Umsetzungsprodukte von Polyisocyanaten mit Hydroxyalkylacrylaten sowie die Umsetzungsprodukte von hydroxylgruppenhaltigen Polyestern oder Polyethern mit Acryl- oder Methacrylsäuree. Diese ungesättigten Acrylharze werden meist im Gemisch mit einem oder mehreren Acrylaten eines Mono-, Di- oder Polyalkohols, wie z. B. Ethyl-, Butyl-, Benzyl-, 2-Ethylhexyl- oder 2-Hydroxypropylacrylat, Ethylenglykoldiacrylat, Propylenglykoldiacrylat, Butandioldiacrylat, Hexamethylen-diacrylat, Trimethylol-propan-trisacrylat oder Pentaerythrit-tetraacrylat, verwendet.

Gegenstand der Erfindung sind auch photopolymerisierbare Systeme, enthaltend a) mindestens eine ethylenisch ungesättigte Verbindung, b) ein definitionsgemässes Gemisch aus A) und B) und gegebenenfalls c) sonstige Zusatzstoffe, wie Inhibitoren, Stabilisatoren, UV-Absorber Füllstoffe, Pigmente, Farbstoffe, Thixotropiemittel und Verlaufshilfsmittel, z. B. Silikonöl.

Als Inhibitoren, welche vor allem während der Herstellung der Systeme durch Mischen der Komponenten vor einer vorzeitigen Polymerisation schützen sollen, werden beispielsweise Hydrochinon, Hydrochinonderivate, p-Methoxyphenyl oder β-Naphthole verwendet. Als UV-Absorber können z. B. solche vom Benztriazol- oder Benzophenontyp eingesetzt werden.

Als Füllstoffe kommen z. B. Kieselsäure, Talkum, Glasfasern, Titandioxid oder Gips in Betracht.

Bevorzugt sind derartige photopolymerisierbare Systeme in den Mengenverhältnissen 99,5 -80 Gew.-% von a) und c) und 0,5 - 20 Gew.-% von b).

Als Komponente a) verwendet man bevorzugt einen Acrylsäureester oder ein Gemisch mehrerer Acrylsäureester.

Insbesondere handelt es sich bei den erfindungsgemässen photopolymerisierbaren Systemen um gefärbte Systeme, die mindestens ein Pigment oder einen Farbstoff enthalten. Solche Systeme finden vor allem als Druckfarben und als Weisslacke Verwendung.

Ein weiteres Verwendungsgebiet der Verbindungen der Formel I ist ihr Einsatz als Photosensibilisatoren in Verbindung mit einem radikalischen oder kationischen Photoinitiator. Radikalische Photoinitiatoren sind solche, die durch Photofragmentierung Radikale bilden. Bekannte Klassen von radikalischen Photoinitiatoren sind z. B. Benzoinether, halogenierte Acetophenone, Benzilketale, Alkoxyacetophenone, Isobutyrophenone (wie sie z. B. in der EP-A-3002, in der DE-OS-2 722 264 oder der EP-A-88 050 beschrieben sind) oder Acylphosphinoxide (wie sie z. B. in der EP-A-7508 beschrieben sind). Kationische Photoinitiatoren sind solche, die bei Bestrahlung Protonen oder Lewis-Säuren abspalten. Bekannte Beispiele hierfür sind aromatische Sulfonium- oder Jodoniumsalze.

Die Kombinationen mit radikalischen Photoinitiatoren werden für die Photopolymerisation ethylenisch ungesättigter Verbindungen verwendet, wie sie vorhin aufgeführt wurden. Die Kombinationen mit kationischen Photoinitiatoren werden zur Photopolymerisation von kationisch polymerisierbaren Verbindungen verwendet. Dies können cyclische Ether oder Thioether sein, Lactone, Lactame oder auch bestimmte Vinylverbindungen. Insbesondere sind Kombinationen mit kationischen Photoinitiatoren für die Strahlenhärtung von Epoxidharzen von Bedeutung.

Grosse Bedeutung haben die erfindungsgemässen Initiatorgemische für die Photohärtung von Druckfarben und weiss pigmentierten Schichten, da die Trocknungszeit des Bindemittels ein massgeblicher Faktor für die Produktionsgeschwindigkeit graphischer Erzeugnisse ist und in der Grössenordnung von Bruchteilen von Sekunden liegen soll. Gut geeignet sind die erfindungsgemässen Initiatoren auch für photohärtbare Systeme zur Herstellung von Druckplatten.

Ein weiteres Einsatzgebiet ist die UV-Härtung von Metallbeschichtungen, beispielsweise bei der Lackierung von Blechen für Tuben, Dosen oder Flaschenverschlüssen, sowie die UV-Härtung von Kunststoffbeschichtungen, beispielsweise von Fussboden- oder Wandbelägen auf PVC-Basis.

Beispiele für die UV-Härtung von Papierbeschichtungen sind die farblose Lackierung von Ettiketten, Schallplatten- Hüllen oder Buchumschlägen.

Die erfindungsgemässen Gemische können auch als Initiatoren zur photochemischen Vernetzung von Polyolefinen verwendet werden.

Hierfür kommen z. B. Polypropylen, Polybuten, Polyisobutylen sowie Copolymerisate wie z. B. Ethylen-Propylen-Copolymere in Frage, vorzugsweise jedoch Polyethylen von niedriger, mittlerer oder hoher Dichte.

Der Zusatz der Photoinitiatoren zu den photopolymerisierbaren Systemen geschieht im allgemeinen durch einfaches Einrühren, da diese Systeme flüssig und gut löslich sind. Es kommt zu einer Lösung oder Dispersion der Initiatoren, wodurch deren gleichmässige Verteilung sowie die Transparenz der Polymerisate gewährleistet ist.

Die Polymerisation erfolgt nach den bekannten Methoden der Photopolymerisation durch Bestrahlung mit Licht das reich an kurzwelliger Strahlung ist. Als Lichtquellen sind z. B. Quecksilbermitteldruck-, -hochdruck- und -niederdruckstrahler, sowie superaktinische Leuchtstoffröhren geeignet, deren Emissionsmaxima im Bereich zwischen 250 und 450 nm liegen.

Bei der photochemischen Vernetzung von Polyolefinen wird der Photoinitiator dem Polyolefin vor oder während der formgebenden Verarbeitung zugesetzt. Die Vernetzung erfolgt durch Bestrahlung des geformten Gegenstandes in fester Form, beispielsweise in Form von Folien oder Fasern.

Die erfindungsgemässen Verbindungen der Formel I eignen sich ferner als Sensibilisatoren für photovernetzbare Polymere der verschiedensten Art. Derartige Polymere werden z. B. zur Herstellung von Druckplatten für das Offsetdruckverfahren, zur Herstellung von Photooffset-Lacken, für die unkonventionelle Photographie, z. B. zur Herstellung von photographischen Bildern mittels Photopolymerisation oder Photovernetzung verwendet. Solche Polymere finden insbesondere Anwendung als sogenannte Photoresists zur Herstellung von gedruckten Schaltungen nach an sich bekannten Methoden. Dabei wird die mit der lichtempfindlichen Schicht versehene Seite der Leiterplatte durch ein das Leiterbild aufweisendes Dianegativ belichtet und dann entwickelt, wobei man die unbelichteten Stellen der Schicht durch Entwicklungsflüssigkeit herauslöst.

Als Polymere können an sich beliebige Materialien verwendet werden, deren Lichtempfindlichkeit (Empfindlichkeit gegenüber aktinischen Strahlen) sich durch den Einsatz der erfindungsgemässen Verbindungen der Formel I erhöhen lässt. Ganz besonders eignen sich die Verbindungen der Formel I als Sensibilisatoren für die in der DE-OS-2 626 769 beschriebenen Polymeren, die als lichtempfindliche Gruppen solche der Formel

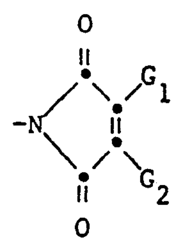

aufweisen, worin $G_1$ und $G_2$ unabhängig voneinander alkyl mit 1 - 4 C-atomen, besonders Methyl, oder $G_1$ und $G_2$ zusammen die Ergänzung zu einem fünf- bis sechsgliedrigen carbocyclischen Ring bedeuten.

Die Verbindungen der Formel I können auf an sich bekannte Weise in die photovernetzbaren Polymeren eingearbeitet werden. Der Gehalt an Verbindungen der Formel I im Polymeren kann je nach anwendungszweck und anzahl der im Polymeren vorhandenen photovernetzbaren Gruppen stark variieren, liegt jedoch im allgemeinen zwischen etwa 0,1 und 20 Gew.-%, bezogen auf das Gewicht des Gemisches.

Die erfindungsgemässen Verbindungen der Formel I in Mischung mit einem organischen Amin eignen sich besonders für wässrige, strahlungshärtbare Beschichtungsmittel, da sie auf Grund des flüssigen Charakters leicht emulgiert und verteilt werden können. Hierdurch wird auch eine gleichmässige Verteilung in einer erzeugten Schicht erzielt. Nach der Bestrahlung einer solchen Schicht erreicht man auch eine gleichmässigere Härtung.

Die nachfolgenden Beispiele erläutern die Erfindung näher.

**Beispiel 1:**

Polyethylenglykol-400-Monoester der Thioxanthon-1-carbonsäure (n ~ 9)
25,63 g (100 mMol) Thioxanthon-1-carbonsäure, 40,00 g (100 mMol) Polyethylenglykol-400 (Fluka, mittleres Molekulargewicht: 380 - 420), 150 ml o-Dichlorbenzol und 138 mg konz. Schwefelsäure werden während 35 Min. zum Rückfluss erhitzt (Badtemperatur 200°C), wobei das Reaktionswasser mittels eines Wasserabscheiders abgetrennt wird. Nach dem Abkühlen wird die Lösung mitwasserfreiem Kaliumcarbonat verrührt, dann filtriert und die Mutterlauge eingedampft, zuerst bei 95°C/17,3 mbar, dann bei 150°C/0,13 mbar. Als Rückstand verbleiben 68,86 g gelbliches, flüssiges Produkt.

UV-Spektrum ($CHCl_3$): $\lambda_{max}$ = 385 nm; ε = 6750
Elementaranalyse: S ber. 5,02 %; gef. 4,91 %.

Analog werden hergestellt:

a) Polyethylenglykol-300-Monoester der Thioxanthon-1-carbonsäure (n ~ 7)
gelbliches, flüssiges Produkt.

UV-Spektrum ($CHCl_3$): $\lambda_{max}$ = 386 nm; ε = 6750
Elementaranalyse: S ber. 5,96 %; 5,89 %.

b) Polyethylenglykol-1000-Monoester der Thioxanthon-1-carbonsäure (n ~ 22)
gelbliches, wachsartig-flüssiges Produkt

UV-Spektrum ($CHCl_3$): $\lambda_{max}$ = 385 nm; ε = 6600
Elementaranalyse: S: ber. 2,61 %; gef. 2,59 %.

**Beispiel 2:**

Polypropylenglykol-400-Monoester der Thioxanthon-1-carbonsäure (n ~ 7)
Die Herstellung erfolgt analog Beispiel 1 mit Polypropylenglykol-400 (Fluka, mittl. Molekulargewicht 380 - 420). Nach 1 Std. am Wasserabscheider wird abgekühlt, mit Kaliumkarbonat und Tonerde verrührt, filtriert und die Mutterlauge eingedampft, zuletzt bei 150°C/0,04 mbar. Als Rückstand wird ein gelbliches Öl erhalten.

UV-Spektrum ($CHCl_3$): $\lambda_{max}$ = 384 nm; ε = 6800
Elementaranalyse: S: ber. 5,03 %; gef. 5,10 %.

**Beispiel 3:**

Polyethylenglykol-400-diester der Thioxanthon-1-carbonsäure (n ~ 9)
25,63 g (100 mMol) Thioxanthon-1-carbonsäure, 20,00 g (50 mMol) Polyethylenglykol-400 (Fluka), 180 ml o-Dichlorbenzol und 128 mg konz. Schwefelsäure werden 2 Std. am Rückfluss unter Wasserabscheidung erhitzt. Die Aufarbeitung analog Beispiel 1 ergibt 46,79 g eines gelblichen, viskosen Öls.

UV-Spektrum ($CHCl_3$): $\lambda_{max}$ = 386 nm; ε = 13 250
Elementaranalyse: S: ber. 7,32 %; gef. 7,32 %.

**Beispiel 4:**

Polyethylenglykol-400-Monoester der Thioxanthon-3-carbonsäure (n ~ 9)
20 g (78 mMol) Thioxanthon-3-carbonsäure, 31,2 g (78 mMol) Polyethylenglykol-400 (Fluka), 200 ml o-Dichlorbenzol und 400 mg Tetrabutylorthotitanat werden während 7 Std. unter Wasserabscheidung am Rückfluss gehalten. Nach dem Abkühlen wird die Lösung mit 40 g wasserfreiem Kaliumcarbonat verrührt, der Niederschlag wird abfiltriert und die Mutterlauge eingeengt, zuletzt bei 150°C/0,4 mbar. Der Rückstand wird mit Toluol und Cyclohexan über Nacht verrührt. Ein gebildeter Niederschlag wird abfiltriert und die Mutterlauge wird zur Trockne eingedampft: Man erhält 33,12 g eines gelben flüssigen Produktes.

UV-Spektrum ($CHCl_3$): $\lambda_{max}$ = 400 nm; ε = 6200.

**Beispiel 5:**

<u>Polyethylenglykol-300-Monoester der 7-Methylthioxanthon-3-carbonsäure (n ~ 7)</u>
15 g (55,2 mMol) 7-Methylthioxanthon-3-carbonsäure und 70 ml Thionylchlorid werden 3 Std. am Rückfluss gehalten. Das Gemisch wird zur Trockne eingedampft und der Rückstand mit 140 ml Toluol und 16,5 g (55,24 mMol) Polyethylenglykol-300 (Fluka) während 3 Std. am Rückfluss gehalten. Nach dem Abkühlen wird die Lösung mit wasserfreiem Kaliumcarbonat verrührt, filtriert und eingedampft. Der teilweise kristalline Rückstand wird mit Toluol/Cyclohexan während 4 Tagen bei 25°C verrührt, der Niederschlag abfiltriert und die Mutterlauge eingedampft. So werden 15,4 g gelbes, flüssiges Produkt erhalten.

UV-Spektrum ($CHCl_3$): $\lambda_{max}$ = 409 nm; $\varepsilon$ = 5800
Elementaranalyse: S: ber. 5,54 %; gef: 5,48 %.

Analog wird hergestellt:

<u>Polyethylenglykol-1000-Monoester der Thioxanthon-3-carbonsäure (n ~ 22)</u>
Gelbe, wachsartig trübe Flüssigkeit.

UV-Spektrum ($CHCl_3$): $\lambda_{max}$ =400 nm; $\varepsilon$ = 5700
Elementaranalyse: S: ber. 2,52 %; gef: 2,51 %.

**Beispiel 6:**

<u>Polyethylenglykol-750-monomethylether-monoester der Thioxanthon-1-carbonsäure (n ~ 16)</u>
3,0 g (11,7 mMol) Thioxanthon-1-carbonsäure, 8,8 g (11,7 mMol) Polyethylenglykol-750-monomethylether (Fluka), 15 ml o-Dichlorbenzol und 70 mg konz. Schwefelsäure werden während 1½ Std. am Rückfluss erhitzt (Badtemperatur 200°C), wobei das Reaktionswasser mittels Wasserabscheider abgetrennt wird. Das Gemisch wird auf etwa 100°C gekühlt, mit Tonerde vermischt, auf 25°C gekühlt und mit 2,5 g Kaliumcarbonat verrührt, filtriert und dann die Mutterlauge eingedampft, zuerst bei 95°C/17,3 mbar, dann bei 150°C/0,13 mbar. Als Rückstand verbleiben 8,0 g eines gelblichen, flüssigen Produktes.

UV-Spektrum ($CHCl_3$): $\lambda_{max}$ = 385 nm; $\varepsilon$ = 6600
Elementaranalyse: S: ber. 4,07 %; gef. 4,37 %.

Analog wird hergestellt:

<u>Polyethylenglykol-550-monomethylether-monoester der Thioxanthon-1-carbonsäure (n ~ 12)</u>
7,6 g gelbliches flüssiges Produkt

UV-Spektrum ($CHCl_3$): $\lambda_{max}$ = 385 nm; $\varepsilon$ = 6200
Elementaranalyse: S: ber. 4,07 %; gef. 4,37 %.

**Beispiel 7:**

38,5 Gewichtsteile Acrylat/Urethan-Oligomer (Actilan Al 18)
19,2 Gewichtsteile N-Vinylpyrrolidon,
19,3 Gewichtsteile $TiO_2$ (RTC-2)
19,2 Gewichtsteile ZnS (Sachtholith HDS)
3,8 Gewichtsteile Butylacetat

werden mit 1 Gew.-% Thioxanthon (bezogen auf die Gesamtrezeptur) vermischt und mit einer 30 µ Rakel elektromotorisch als Film auf eine Glasplatte aufgezogen und getrocknet.

Die UV-Härtung der Filme wird mit einer (Tabelle 1) Standard-Quecksilberdampflampe (QC-Processor, RPC) oder mit einer (Tabelle 2) elektrodenlosen Quecksilberdampflampe (Typ D, Fusion System Inc.) vorgenommen. Die Glasplatten werden mit einer Geschwindigkeit von 10 m/min an den Lampen vorbeigeführt.

Die Ergebnisse sind in den nachfolgenden Tabellen 1 und 2 angegeben. YI heisst Yellowness Index.

**Tabelle 1**

| Thioxanthonsäureester | Trockenfilmstärke | Wischfest (WF) nach 10 m/min | Pendelhärte bei WF (Sek.) | Vergilbung (YI) bei WF. |
|---|---|---|---|---|
| gemäss Beispiel 1 | 14 | 3 x | 40 | - 1,9 |
| gemäss Beispiel 1a | 12 | 2 x | 36 | - 1,6 |
| gemäss Beispiel 3 | 15 | 3 x | 42 | - 1,2 |

**Tabelle 2:**

| Thioxanthonsäureester | Trockenfilmstärke | Wischfest (WF) nach 10 m/min | Pendelhärte bei bei WF (Sek.) | Vergilbung (YI) bei WF. |
|---|---|---|---|---|
| gemäss Beispiel 1 | 15 | 2 x | 47 | - 1,5 |
| gemäss Beispiel 1a | 18 | 2 x | 42 | - 0,5 |
| gemäss Beispiel 3 | 15 | 2 x | 53 | - 0,5 |

**Beispiel 8:**

100 Gew.-Teile einer 55-%-igen wässrigen Dispersion eines ungesättigten Polyesterharzes (ROSKYDAL® W 850, Bayer AG) werden mit 0,25 Gew.-Teilen des Thioxanthoncarbonsäureesters aus Beispiel 1 und 1,5 Gew.-Teilen N-Methyldiethanolamin vermischt und mit einer 200 μ-Rakel elektromotorisch als Film auf eine Glasplatte aufgetragen. Die Härtung des Films geschieht durch Bestrahlung mit einer Standard-Quecksilberdampflampe im QC-Processor. Die Glasplatte wird dabei mit einer Geschwindigkeit von 10 m/min an der Lampe vorbeigeführt.

Nach 8 Durchgängen ist der Film wischfest. Die Oberfläche des Filmes ist glatt und gleichmässig. Wird in derselben Rezeptur anstelle des Thioxanthoncarbonsäureesters Isopropylthioxanthon verwendet, so wird die Filmoberfläche unregelmässig. Man erkennt Krater und Stippen sowie viele Nadelstiche.

## Patentansprüche

1. Flüssige Thioxanthoncarbonsäureester der Formel I und flüssige Mischungen von Thioxanthoncarbonsäureestern der Formel I

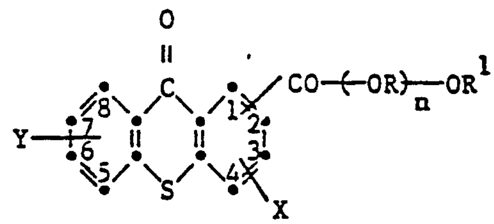

(I) ,

worin

X H, Halogen, $NO_2$, COOH, gegebenenfalls alkylsubstituiertes Amino, $C_1$-$C_{20}$-Alkyl, $C_1$-$C_{20}$-Alkoxy, $C_1$-$C_{20}$-Alkylthio, $C_1$-$C_{20}$-Alkylsulfinyl, $C_1$-$C_{20}$-Alkylsulfonyl-, unsubstituiertes oder substituiertes Phenoxy, Phenylthio, Phenylsulfinyl oder Phenylsulfonyl, oder -$COOR^a$ ist, worin $R^a$ für $C_1$-$C_6$-Alkyl, Cyclopentyl, Cyclohexyl, Phenyl, ($C_1$-$C_4$-Alkyl)-Phenyl, Benzyl oder ($C_1$-$C_4$-Alkyl)-Benzyl steht,

Y unabhängig die gleiche Bedeutung wie X hat,

n für eine Zahl von 4 bis 25 steht,

R lineares oder verzweigtes $C_2$-$C_6$-Alkylen bedeutet, und

$R^1$ für H, $C_1$-$C_{12}$-Alkyl oder einen Rest der Formel II

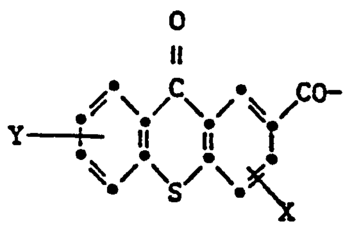

(II)

steht, wobei n eine Zahl von 7 - 25 ist wenn R Ethylen und $R^1$ H bedeuten, und n eine Zahl von 11 - 25 ist, wenn R für Ethylen und $R^1$ für Methyl stehen.

2. Ester und Mischungen gemäss Anspruch 1, dadurch gekennzeichnet, dass X für H steht.

3. Ester und Mischungen gemäss Anspruch 1, dadurch gekennzeichnet, dass Y für H oder $C_1$-$C_6$-Alkyl, besonders Methyl steht.

4. Ester und Mischungen gemäss Anspruch 1, dadurch gekennzeichnet, dass R $C_2$-$C_4$-Alkylen ist.

5. Ester und Mischungen gemäss Anspruch 4, dadurch gekennzeichnet, dass R Ethylen, 1,2- oder 1,3-Propylen ist.

6. Ester und Mischungen gemäss Anspruch 1, dadurch gekennzeichnet, dass $R^1$ $C_1$-$C_6$-Alkyl ist.

7. Ester und Mischungen gemäss Anspruch 1, dadurch gekennzeichnet, dass $R^1$ Wasserstoff, Methyl oder Ethyl ist.

8. Ester und Mischungen gemäss Anspruch 1, dadurch gekennzeichnet, dass $R^1$ ein Rest der Formel II ist.

9. Ester und Mischungen gemäss Anspruch 1, dadurch gekennzeichnet, dass Y in 7-Stellung gebunden ist.

10. Ester und Mischungen gemäss Anspruch 1, dadurch gekennzeichnet, dass die Gruppe $-CO-(-OR-)_n-OR^1$ in 1- oder 3-Stellung gebunden ist.

11. Ester und Mischungen gemäss Anspruch 1, dadurch gekennzeichnet, dass n für eine Zahl von 8 bis 20, bevorzugt 8 bis 14 steht, wenn $R^1$ H oder einen Rest der Formel II bedeutet, oder dass n für eine Zahl von 11 bis 20, vorzugsweise 12 bis 18 steht, wenn $R^1$ Alkyl bedeutet.

12. Ester und Mischungen gemäss Anspruch 1, dadurch gekennzeichnet, dass R Ethylen ist, $R^1$ H oder einen Rest der Formel II bedeutet und n eine Zahl von 8 bis 14 ist, oder dass R Ethylen und $R^1$ Methyl sind und n eine Zahl von 12 bis 18 ist.

13. Photopolymerisierbares System enthaltend
a) mindestens eine ethylenisch ungesättigte Verbindung,
b) mindestens eine Verbindung der Formel I des Anspruchs 1,
c) mindestens ein organisches Amin und gegebenenfalls
d) sonstige Zusatzstoffe.

14. Photopolymerisierbares System gemäss Anspruch 13, das ein wässriges Beschichtungsmittel ist.

15. Photopolymerisierbares System enthaltend
a) ein photovernetzbares Polymer und
b) mindestens eine Verbindung der Formel I des Anspruchs 1.

16. System gemäss Anspruch 16, enthaltend 0,1 bis 20 Gew.-% der Komponente b).

## Claims

1. A liquid thioxanthonecarhoxylic acid ester of the formula I or a liquid mixture of thioxanthonecarboxylic acid esters of the formula I

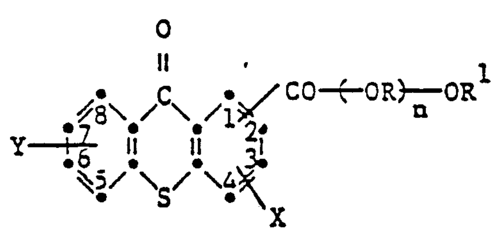

(I)

X is H, halogen, $NO_2$, COOH, unsubstituted or alkyl-substituted amino, $C_1$-$C_{20}$alkyl, $C_1$-$C_{20}$alkoxy, $C_1$-$C_{20}$alkylthio, $C_1$-$C_{20}$alkylsulfinyl, $C_1$-$C_{20}$alkylsulfonyl, unsubstituted or substituted phenoxy, phenylthio, phenylsulfinyl or phenylsulfonyl, or is $-COOR^a$, in which $R^a$ is $C_1$-$C_6$alkyl, cyclopentyl, cyclohexyl, phenyl, ($C_1$-$C_4$alkyl)phenyl, benzyl or ($C_1$-$C_4$alkyl)benzyl,
Y has in each case independently the same meaning as X,
n is a number from 4 to 25,
R is linear or branched $C_2$-$C_6$alkylene and
$R^1$ is H, $C_1$-$C_{12}$alkyl or a radical of the formula II

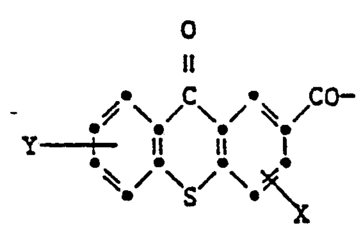

(II)

where, if R is ethylene and $R^1$ is H, n is a number from 7 to 25 and, if R is ethylene and $R^1$ is methyl, n is a number from 11 to 25.

2. An ester or mixture according to claim 1, wherein X is H.

3. An ester or mixture according to claim 1, wherein Y is H or $C_1$-$C_6$alkyl, in particular methyl.

4. An ester or mixture according to claim 1, wherein R is $C_2$-$C_4$alkylene.

5. An ester or mixture according to claim 4, wherein R is ethylene, 1,2-propylene or 1,3-propylene.

6. An ester or mixture according to claim 1, wherein $R^1$ is $C_1$-$C_6$alkyl.

7. An ester or mixture according to claim 1, wherein $R^1$ is hydrogen, methyl or ethyl.

8. An ester or mixture according to claim 1, wherein $R^1$ is a radical of the formula II.

9. An ester or mixture according to claim 1, wherein Y is bonded in the 7-position.

10. An ester or mixture according to claim 1, wherein the -CO-(OR)-$_n$-OR$^1$ group is bonded in the 1- or 3-position.

11. An ester or mixture according to claim 1, wherein n is a number from 8 to 20, preferably from 8 to 14, if $R^1$ is H or a radical of the formula II, or n is a number from 11 to 20, preferably from 12 to 18, if $R^1$ is alkyl.

12. An ester or mixture according to claim 1, wherein R is ethylene, $R^1$ is H or a radical of the formula II and n is a number from 8 to 14, or R is ethylene and $R^1$ is methyl and n is a number from 12 to 18.

13. A photopolymerisable system containing
a) at least one ethylenically unsaturated compound,
b) at least one compound of the formula I of claim 1,
c) at least one organic amine and, if appropriate,
d) other additives.

14. A photopolymerisable system according to claim 13, which is an aqueous coating composition.

15. A photopolymerisable system containing
a) a photocrosslinkable polymer and
b) at least one compound of the formula I of claim 1.

16. A system according to claim 16, which contains 0.1 to 20 % by weight of component b).

## Revendications

1. Esters liquides d'acides thioxanthonecarboxyliques de formule I et mélanges liquides d'esters d'acides thioxanthone-carboxyliques répondant à la formule I:

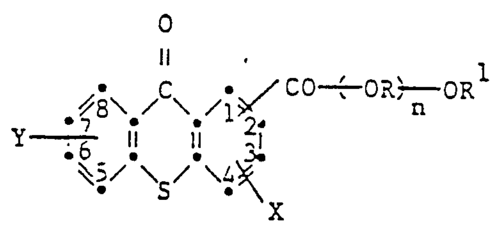

(I)

dans laquelle:

X représente H, un halogène, $NO_2$, COOH, un amino éventuellement porteur d'un alkyle, un alkyle en $C_1$-$C_{20}$, un alcoxy en $C_1$-$C_{20}$, un alkylthio en $C_1$-$C_{20}$, un alkylsulfinyle en $C_1$-$C_{20}$, un alkylsulfonyle en $C_1$-$C_{20}$, un radical phénoxy, phénylthio, phénylsulfinyle ou phénylsulfonyle substitué ou non, ou un radical -COOR$^a$ dans lequel R$^a$ représente un alkyle en $C_1$-$C_6$, un cyclopentyle, un cyclohexyle, un phényle, un ($C_1$-$C_4$-alkyl)-phényle, un benzyle ou un ($C_1$-$C_4$-alkyl)-benzyle,

Y a la signification qui vient d'être donnée pour X mais indépendamment de X,

n désigne un nombre de 4 à 25,

R représente un alkylène en $C_2$-$C_6$ linéaire ou ramifié, et

$R^1$ représente H, un alkyle en $C_1$-$C_{12}$ ou un radical de formule II:

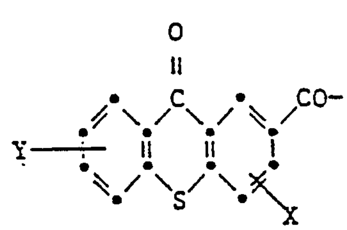

(II),

n désignant un nombre de 7 à 25 dans le cas où R représente un radical éthylène et $R^1$ l'hydrogène, et n désignant un nombre de 11 à 25 dans le cas où R représente un radical éthylène et $R^1$ un radical méthyle.

2. Esters et mélanges selon la revendication 1, caractérisés en ce que X représente H.

3. Esters et mélanges selon la revendication 1, caractérisés en ce que Y représente H ou un alkyle en $C_1$-$C_6$, plus particulièrement un méthyle.

4. Esters et mélanges selon la revendication 1, caractérisés en ce que R représente un alkylène en $C_2$-$C_4$.

5. Esters et mélanges selon la revendication 4, caractérisés en ce que R représente un radical éthylène, propyléne-1,2 ou propyléne-1,3.

6. Esters et mélanges selon la revendication 1, caractérisés en ce que $R^1$ représente un alkyle en $C_1$-$C_6$.

7. Esters et mélanges selon la revendication 1, caractérisés en ce que $R^1$ représente l'hydrogène, un méthyle ou un éthyle.

8. Esters et mélanges selon la revendication 1, caractérisés en ce que $R^1$ représente un radical de formule II.

9. Esters et mélanges selon la revendication 1, caractérisés en ce que Y se trouve à la position 7.

10. Esters et mélanges selon la revendication 1, caractérisés en ce que le radical $-CO-(-OR-)_n-OR^1$ se trouve à la pposition 1 ou à la position 3.

11. Esters et mélanges selon le revendication 1, caractérisés en ce que n désigne un nombre de 8 à 20, de préférence de 8 à 14, dans le ces où $R^1$ représente l'hydrogène ou un radical de formule II, ou en ce que n désigne un nombre de 11 à 20, de préférence de 12 à 18, dans le cas où $R^1$ représente un alkyle.

12. Esters et mélanges selon le revendication 1, caractérisés en ce que R représente un radical éthylène, $R^1$ l'hydrogène ou un radical de formule II et n un nombre de 8 à 14, ou en ce que R représente un radical éthylène, $R^1$ un radical méthyle et n un nombre de 12 à 18.

13. Composition photopolymérisable qui contient:
a) au moins un composé éthylénique,
b) au moins un composé de formule I selon la revendication 1,
c) au moins une amine organique, et éventuellement
d) d'autres additifs.

14. Composition photopolymérisable selon la revendication 13, qui est un produit de revêtement aqueux.

15. Composition photopolymérisable qui contient:
a) un polymère photoréticulable et
b) au moins un composé de formule I selon la revendication 1.

16. Composition selon la revendication 15 qui contient de 0,1 à 20 % en poids de la composante b).